# EUROPEAN PATENT SPECIFICATION

(11) **EP 1 463 452 B1**
(45) Date of publication and mention of the grant of the patent: **18.05.2011**
(21) Application number: 02805461.7
(22) Date of filing: 20.12.2002
(51) Int. Cl.: A61B 17/06

(54) **SURGICAL INSTRUMENT**
CHIRURGISCHES INSTRUMENT
INSTRUMENT CHIRURGICAL

(30) Priority: 20.12.2001 AU PR969701
(43) Date of publication of application: 06.10.2004
(73) Proprietor: Covidien AG, 8212 Neuhausen am Rheinfall (CH)
(72) Inventor: PETROS, Peter, South Perth 6151 Western Australia (AU)
(74) Representative: Soames, Candida Jane
(86) International application number: PCT/IB2002/005783
(87) International publication number: WO 2003/053252

(56) References cited:
- WO-A-01/78609
- WO-A-01/93656
- GB-A- 309 633
- SU-A- 740 240
- US-A- 5 149 329
- US-A- 5 222 977
- US-A- 5 250 054
- US-A- 5 899 909

## Description

### BACKGROUND

### 1. Technical Field

This invention relates to a surgical instrument intended for use in location of a filament in the body of a patient, and more particularly, to a surgical instrument for use in placement of a support in the body to relieve vaginal prolapse.

### 2. Background of Related Art

Normal vaginal delivery exposes the female pelvic floor to muscle and connective tissue trauma which, in some cases, can result in pelvic floor retraction and pelvic organ prolapse. Vaginal prolapse is a result of weakening of connective tissue support to the vaginal vault apex. One of the most common surgical techniques used to correct vaginal prolapse includes tying the upper part of the vagina to a connective tissue condensation stretched from both sides of the sacrum. Placement and securement of a support in this area of the body is often difficult.

WO 01/78609 A2 discloses a combination of features falling within the scope of the pre-characterizing portion of Claim 1. Further prior art disclosure is found in WO 01/93656 A2, US-A-5 899 909, SU 740 240 A, US-A-5 250 054 and US-A-5 149 329.

### SUMMARY

According to the present invention, there is provided a surgical instrument intended for use in location of a filament in the body of a patient, as defined in claim 1. Preferred embodiments are defined in the dependent claims.

According to a preferred feature of the invention the eye has an axial dimension which is greater than the transverse dimension. According to a preferred feature of one embodiment, the slot has a width which is less than the axial dimension of the eye.

According to a preferred feature of the instrument the portion proximate the second end comprises approximately one quarter to one half of the axial extant of the shaft.

According to a preferred feature of the instrument the handle members have a generally triangular shape. According to a preferred feature of the instrument the plane which includes the handle members is generally perpendicular to the portion of the shaft proximate the second end.

### BRIEF DESCRIPTION OF THE DRAWINGS

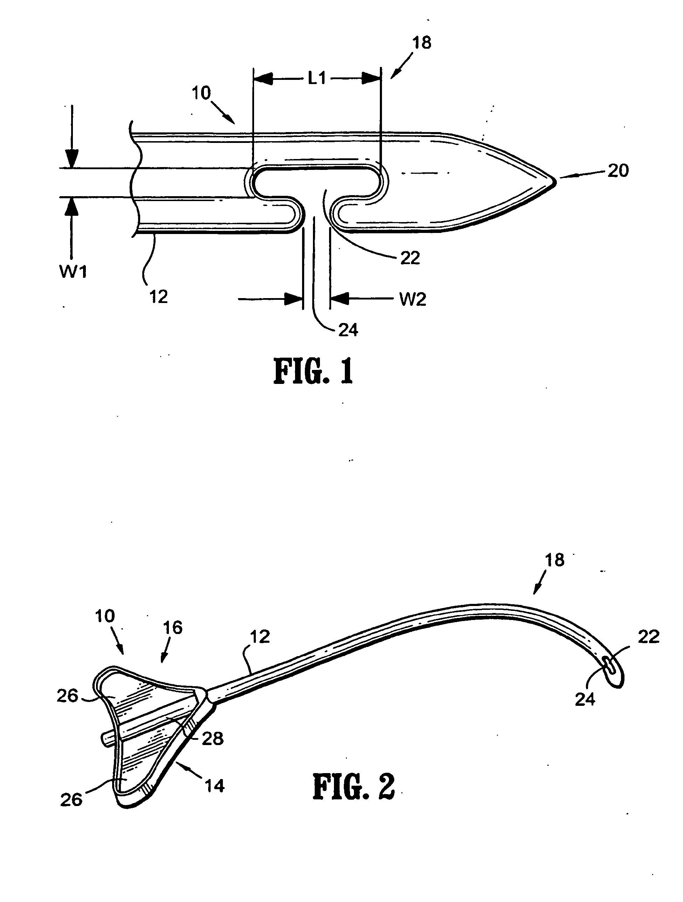

To enable a better understanding of the present invention, and to show how the same may be carried into effect, reference will now be made, by way of example only, to the accompanying drawings, in which:-
Figure 1 is an isometric view of a surgical instrument according to the embodiment; and
Figure 2 is an enlarged view of the other end of the shaft incorporating the eye.

### DETAILED DESCRIPTION

The description is directed to an embodiment of a surgical instrument 10 which can be utilised for the location of a filament comprising a tape formed of a suitable material into the body. An application for the surgical instrument relates to the insertion of a tape from the tissue surrounding the vagina into the vaginal cavity for the purposes of supplementing or replacing support for the vagina in the event of vaginal prolapse.

Surgical instrument 10 includes a shaft 12 having a handle 14 at a first end16. A second end 18 of the shaft 12 is convergent to provide a substantially pointed end 20. The outer most third (approximately) of the shaft 12 is curved through substantially 90 degrees. The second end 18 is provided with an eye 22 adjacent to the second end 18 where the eye 22 has an axial dimension L1 which is greater than the transverse dimension W1. In addition the eye 22 is provided with a slot 24 which extends transversely from the eye 22 to the outer wall of the shaft 12. The width W2 of the slot 24 is less than the axial dimension L1 of the eye 22 and as a result the eye 22 and slot 24 jointly define an opening which extends transversely across the shaft 12 which has a generally keyhole like cross-sectional configuration.

The handle 14 comprises a pair of lateral portions or "wings" 26 which extend to either side of a central tubular boss 28 which receives the shaft 12.
The plane of the handle 14 is generally perpendicular to the plane of the curved shaft 12.
The surgical instrument 10 according to the embodiment provides a means whereby a length of tape can be accurately located in position in the patient's body when used in relation to an operation involving resolution of vaginal prolapse.

In use the shaft 12 is inserted into the body through an incision and is caused to pass through the body and to penetrate the vaginal wall through a preformed incision. Once in that location the eye 22 can be accessed through the vagina to allow insertion of a length of tape through the eye 22. With the withdrawal of the shaft 22 from the body, the tape can then be drawn through the body to extend from the vagina to the exterior of the body. This process is then repeated to the opposite side of the body to provide access between the eye 22 of the shaft and the remaining length of tape received within the vagina to enable that portion of the tape to be extended through the body. The ends of the tape may then be fixed to tissue within the body in order to supplement or replace pre-existing support.

The curvature of the shaft 12 facilitates manipulation and location of the eye 22 while the configuration of the eye 22 readily enables the tape to be inserted in position in the eye 22 and without undue dexterity being required.

Throughout the specification, unless the context requires otherwise, the word "comprises" or variations such as "comprises" or "comprising", will be understood to imply the inclusion of a stated integer or group of integers but not the exclusion of any other integer or group of integers.

It should be appreciated that the scope of the claims is not limited to the particular embodiment described

## Claims

1. A surgical instrument (10) for use in location of a filament in the body of a patient, said instrument comprising a rigid shaft (12) having a handle (14) at a first end (16), a second end (18) of the shaft being formed with an eye (22) adjacent to the second end (18), wherein:
said eye extends in an axial direction of the second end, and is provided with a slot (24) extending orthogonally from the eye (22), from substantially the centre of the eye (22) in the axial direction, to open laterally to one side of the shaft (12) such that the opening defined by the eye (22) and the slot (24) has a generally T-shaped cross-sectional configuration, the eye being dimensioned to receive said filament, and
the shaft (12) is curved in a portion proximate the second end (18); and
**characterized in that**:
the handle (14) comprises a pair of handle members (26, 26) which extend transversely from the first end (16) of the shaft in opposed relation to each other.

2. A surgical instrument as claimed in claim 1 wherein the shaft (12) is convergent.

3. A surgical instrument as claimed in claim 1 or 2. wherein the eye (22) has an axial dimension (L1) which is greater than the transverse dimension (W1).

4. A surgical instrument as claimed in claim 3, wherein the slot has a width (W2) which is less than the axial dimension (L1) of the eye.

5. A surgical instrument as claimed in any preceding claim, wherein the curved portion proximate the second end (18) comprises approximately one quarter to one half of the axial extent of the shaft (12).

6. A surgical instrument as claimed in any preceding claim, wherein the handle members (26, 26) have a generally triangular shape.

7. A surgical instrument as claimed in any preceding claim, wherein the plane which includes the handle members (26, 26) is generally perpendicular to the plane which contains the curved portion of the shaft (12) proximate the second end (18).

## Patentansprüche

1. Chirurgisches Instrument (10) zur Verwendung an einem Ort eines Filaments im Körner eines Patienten, wobei das Instrument einen festen Schaft (12) mit einem -Griff (14) an einem ersten Ende (16) umfasst, wobei ein zweites Ende (18) des Schafts mit einem Auge (22) in der Nähe des zweiten Endes (18) ausgebildet ist, wobei:
sich das Auge in einer axialen Richtung des zweiten Endes erstreckt und mit einem Schlitz (24) versehen ist, der sich orthogonal von dem Auge (22) im Wesentlichen von der Mitte des Auges (22) in der axialen Richtung erstreckt, um sich seitlich zu einer Seite des Schafts (12) zu öffnen, so dass die durch das Auge (22) und den Schlitz (24) definierte Öffnung eine allgemein T-förmige Querschnittsform hat, wobei das Auge dazu bemessen ist, das Filament aufzunehmen, und der Schaft (12) in einem Abschnitt proximal von dem zweiten Ende (18) gekrümmt ist; und
**dadurch gekennzeichnet, dass**:
der Griff (14) ein Paar Griffelemente (26, 26) umfasst, die sich quer von dem ersten Ende (16) des Schafts in einander gegenüberliegender Beziehung erstrecken.

2. Chirurgisches Instrument nach Anspruch 1, wobei der Schaft (12) konvergierend ist.

3. Chirurgisches Instrument nach Anspruch 1 oder 2, wobei das Auge (22) eine axiale Dimension (L1) aufweist, die größer als die Querdimension (W1) ist.

4. Chirurgisches Instrument nach Anspruch 3, wobei der Schlitz eine Breite (W2) aufweist, die kleiner als die axiale Dimension (L1) des Auges ist.

5. Chirurgisches Instrument nach einem vorhergehenden Anspruch, wobei der gekrümmte Abschnitt proximal von dem zweiten Ende (18) in etwa ein Viertel bis eine Hälfte der axialen Ausdehnung des Schafts (12) umfasst.

6. Chirurgisches Instrument nach einem der vorhergehenden Ansprüche, wobei die Griffelemente (26, 26) eine allgemeine Dreiecksform aufweisen.

7. Chirurgisches Instrument nach einem der vorhergehenden Ansprüche, wobei die Ebene, die die Griffelemente (26, 26) enthält, im Allgemeinen senkrecht zu der Ebene liegt, welche die gekrümmten Abschnitte des Schafts (12) proximal von dem zweiten Ende (18) enthält.

## Revendications

1. Instrument chirurgical (10) à utiliser pour placer un filament dans le corps d'un patient, ledit instrument comprenant un arbre rigide (12) ayant une poignée (14) à une première extrémité (16), une seconde extrémité (18) de l'arbre présentant un oeillet (22) adjacent à la seconde extrémité (18), dans lequel :
ledit sillet s'étend dans une direction axiale de la seconde extrémité et présente une fente (24) s'étendant orthogonalement de l'oeillet (22), sensiblement du centre de feuillet (22) dans la direction axiale, pour s'ouvrir latéralement vers un côté de l'arbre (12) de sorte que l'ouverture définie par l'oeillet (22) et la fente (24) possède une configuration en section transversale généralement en forme de T, l'oeillet étant dimensionné pour recevoir ledit filament, et
l'arbre (12) est courbé dans une portion à proximité de la seconde extrémité (18) ; et
**caractérisé en ce que** :
la poignée (14) comprend une plaire d'éléments de poignée (26, 26) qui s'étendent transversalement depuis la première extrémité (16) de l'arbre dans une relation opposée l'un à l'autre.

2. Instrument chirurgical selon la revendication 1, dans lequel l'arbre (12) est convergent.

3. Instrument chirurgical selon la revendication 1 ou 2, dans lequel l'oeillet (22) possède une dimension axiale (L1) qui est plus grande que la dimension transversale (W1).

4. Instrument chirurgical selon la revendication 3, dans lequel la fente a une largeur (W2) qui est plus petite que la dimension axiale (L1) de l'oeillet.

5. Instrument chirurgical selon l'une quelconque des revendications précédentes, dans lequel la portion courbée à proximité de la seconde extrémité (18) comprend un quart à une moitié de l'étendue axiale de l'arbre (12).

6. Instrument chirurgical selon l'une quelconque des revendications précédentes, dans lequel les éléments de poignée (26, 26) ont une forme généralement triangulaire.

7. Instrument chirurgical selon l'une quelconque des revendications précédentes, dans lequel le plan qui comporte les éléments de poignée (26, 26) est généralement perpendiculaire au plan qui contient la partie courbée de l'arbre (12) à proximité de la seconde extrémité (18).
